# EUROPEAN PATENT APPLICATION

(11) **EP 1 193 313 A1**
(43) Date of publication of application: **03.04.2002**
(21) Application number: 00937241.8
(22) Date of filing: 15.06.2000
(51) Int. Cl.: C12N 15/48, C12Q 1/68, C12Q 1/70, G01N 33/569, G01N 33/50

(54) **METHOD FOR DETERMINING HIV-1 SUBTYPE**

(30) Priority: 15.06.1999 JP 16773699; 01.02.2000 JP 2000023581
(71) Applicant: OTSUKA PHARMACEUTICAL CO., LTD., Chiyoda-ku Tokyo 101-8535 (JP); Keio University, Tokyo 153-0062 (JP)
(72) Inventor: KATO, Shingo, Tokyo 177-0031 (JP); KOBAYASHI, Yoshio, Tokyo 157-0066 (JP); HIRAISHI, Yoshiyuki, Tokyo 203-0054 (JP); SHIMIZU, Kayoko, Yokohama-shi Kanagawa 224-0037 (JP); SUGITA, Tetsuyoshi, Tokyo 164-0012 (JP)
(74) Representative: HOFFMANN - EITLE
(86) International application number: JP0003896
(87) International publication number: WO0077219

(57) **Abstract**

The present invention relates to a method for determining HIV-1 subtypes, characterized by comprising the steps of amplifying nucleic acid using as a target sequence a portion of a nucleotide sequence of the env gene of HIV-1, where at least one of the 5' terminal and 3' terminal nucleotide sequences is different depending on the HIV-1 subtype, and detecting the subtype depending on whether or not the nucleic acid has been amplified. The present invention also relates to a kit for determining HIV-1 subtypes, comprising primer pairs in which a target sequence is a portion of a nucleotide sequence of the env gene of HIV-1, where at least one of the 5' terminal and 3' terminal nucleotide sequences is different depending on the subtype.

SEQUENCE LISTING

<110> OTSUKA PHARMACEUTICAL CO., LTD.
<110> KEIO UNIVERSITY

<120> A METHOD FOR HIV-1 SUBTYPING

<130> P00-18

<140>
<141>

<150> JP P11-167736
<151> 1999-06-15

<150> JP P2000-23581
<151> 2000-02-01

<160> 34

<170> PatentIn Ver. 2.1

<210> 1
<211> 20
<212> DNA
<213> Artificial Sequence

<220>
<223> Description of Artificial Sequence: synthetic DNA

<400> 1
ctcctgaggg gttagcaaag                                                20

<210> 2
<211> 20

<212> DNA
<213> Artificial Sequence

<220>
<223> Description of Artificial Sequence: synthetic DNA

<400> 2
ctgtgcatta caatttctgg                                                    20


<210> 3
<211> 20
<212> DNA
<213> Artificial Sequence

<220>
<223> Description of Artificial Sequence: synthetic DNA

<400> 3
ctcctgaggg tggttgaaag                                                    20


<210> 4
<211> 20
<212> DNA
<213> Artificial Sequence

<220>
<223> Description of Artificial Sequence: synthetic DNA

<400> 4
aaatggcagt ctagcagaag                                                    20


<210> 5
<211> 20

<212> DNA
<213> Artificial Sequence

<220>
<223> Description of Artificial Sequence: synthetic DNA

<400> 5
gcaatagaaa aattctcctc                                                    20


<210> 6
<211> 20
<212> DNA
<213> Artificial Sequence

<220>
<223> Description of Artificial Sequence: synthetic DNA

<400> 6
acagtagaaa aattcccctc                                                    20


<210> 7
<211> 20
<212> DNA
<213> Artificial Sequence

<220>
<223> Description of Artificial Sequence: synthetic DNA

<400> 7
gcaatagaaa aattcccctc                                                    20


<210> 8
<211> 20

<212> DNA
<213> Artificial Sequence

<220>
<223> Description of Artificial Sequence: synthetic DNA

<400> 8
cacagtacaa tgcacacatg                                                    20

<210> 9
<211> 20
<212> DNA
<213> Artificial Sequence

<220>
<223> Description of Artificial Sequence: synthetic DNA

<400> 9
cacagtacaa tgtacacatg                                                    20

<210> 10
<211> 20
<212> DNA
<213> Artificial Sequence

<220>
<223> Description of Artificial Sequence: synthetic DNA

<400> 10
aaatggtagc ctagcagaag                                                    20

<210> 11
<211> 20

<212> DNA
<213> Artificial Sequence

<220>
<223> Description of Artificial Sequence: synthetic DNA

<400> 11
gaatggcagt ttagcagaag                                                    20


<210> 12
<211> 20
<212> DNA
<213> Artificial Sequence

<220>
<223> Description of Artificial Sequence: synthetic DNA

<400> 12
gtcaaatggc agtttagcag                                                    20


<210> 13
<211> 22
<212> DNA
<213> Artificial Sequence

<220>
<223> Description of Artificial Sequence: synthetic DNA

<400> 13
ctcctgagga tggtgcaaat tt                                                 22


<210> 14
<211> 22

<212> DNA
<213> Artificial Sequence

<220>
<223> Description of Artificial Sequence: synthetic DNA

<400> 14
ctcctgagga tggtttaaaa at                                                22

<210> 15
<211> 22
<212> DNA
<213> Artificial Sequence

<220>
<223> Description of Artificial Sequence: synthetic DNA

<400> 15
ctcctgagga tgagttaaat tt                                                22

<210> 16
<211> 20
<212> DNA
<213> Artificial Sequence

<220>
<223> Description of Artificial Sequence: synthetic DNA

<400> 16
tcctgcagat gagttaaagg                                                   20

<210> 17
<211> 20

<212> DNA
<213> Artificial Sequence

<220>
<223> Description of Artificial Sequence: synthetic DNA

<400> 17
tcctgaggat ggtttaaagg                                                    20

<210> 18
<211> 20
<212> DNA
<213> Artificial Sequence

<220>
<223> Description of Artificial Sequence: synthetic DNA

<400> 18
aatttctggg tcccctcctg                                                    20

<210> 19
<211> 20
<212> DNA
<213> Artificial Sequence

<220>
<223> Description of Artificial Sequence: synthetic DNA

<400> 19
aatttctaga tctcctcctg                                                    20

<210> 20
<211> 20

<212> DNA
<213> Artificial Sequence

<220>
<223> Description of Artificial Sequence: synthetic DNA

<400> 20
ctgttaaatg gcagtctagc                                                    20

<210> 21
<211> 22
<212> DNA
<213> Artificial Sequence

<220>
<223> Description of Artificial Sequence: synthetic DNA

<400> 21
ctcaactact gttaaatggt ag                                                 22

<210> 22
<211> 20
<212> DNA
<213> Artificial Sequence

<220>
<223> Description of Artificial Sequence: synthetic DNA

<400> 22
ctgttaaatg gcagcctagc                                                    20

<210> 23
<211> 20

<212> DNA
<213> Artificial Sequence

<220>
<223> Description of Artificial Sequence: synthetic DNA

<400> 23
ctgttaaatg gcagtttagc                                                    20

<210> 24
<211> 20
<212> DNA
<213> Artificial Sequence

<220>
<223> Description of Artificial Sequence: synthetic DNA

<400> 24
ctgttaaatg gtagtctagc                                                    20

<210> 25
<211> 20
<212> DNA
<213> Artificial Sequence

<220>
<223> Description of Artificial Sequence: synthetic DNA

<400> 25
   aatttctaga tcccctcctg                                                    20

<210> 26
<211> 20

<212> DNA
<213> Artificial Sequence

<220>
<223> Description of Artificial Sequence: synthetic DNA

<400> 26
aatttctagg tcccctcctg                                                    20

<210> 27
<211> 20
<212> DNA
<213> Artificial Sequence

<220>
<223> Description of Artificial Sequence: synthetic DNA

<400> 27
ctcctgagga gttagcaaag                                                    20

<210> 28
<211> 22
<212> DNA
<213> Artificial Sequence

<220>
<223> Description of Artificial Sequence: synthetic DNA

<400> 28
cacaattaaa actgtgcatt ac                                                 22

<210> 29
<211> 22

<212> DNA
<213> Artificial Sequence

<220>
<223> Description of Artificial Sequence: synthetic DNA

<400> 29
ttgttttatt agggaagtgt tc                                                22

<210> 30
<211> 24
<212> DNA
<213> Artificial Sequence

<220>
<223> Description of Artificial Sequence: synthetic DNA

<400> 30
ctctacaatt aaaatgatgc attg                                              24

<210> 31
<211> 20
<212> DNA
<213> Artificial Sequence

<220>
<223> Description of Artificial Sequence: synthetic DNA

<400> 31
ttctcctcta caattaaagc                                                   20

<210> 32
<211> 22

<212> DNA
<213> Artificial Sequence

<220>
<223> Description of Artificial Sequence: synthetic DNA

<400> 32
ttattgtttt attagggaag tg                                                22

<210> 33
<211> 22
<212> DNA
<213> Artificial Sequence

<220>
<223> Description of Artificial Sequence: synthetic DNA

<400> 33
tgcattgtaa tttctagatc tc                                                22

<210> 34
<211> 20
<212> DNA
<213> Artificial Sequence

<220>
<223> Description of Artificial Sequence: synthetic DNA

<400> 34
tgatgcattg taatttctag                                                   20

## Description

### TECHNICAL FIELD

The present invention relates to a method for determining HIV-1 subtypes, and a kit for determining HIV-1 subtypes.

### BACKGROUND ART

The virus causing acquired immune deficiency syndrome (AIDS) is the human immunodeficiency virus (HIV), of which type 1 (HIV-1) and type 2 (HIV-2) are known. Most cases involve HIV-1, for which various subtypes have been discovered.

Determining the HIV-1 subtype in infected individuals is important for assessing the route of infection and the reliability of virological test results (particularly the drug resistance based on genotype or the determination of plasma HIV-1 RNA concentration). HIV-1 subtypes are generally determined through the sequencing of specific regions of the virus genome and phylogenetic analysis of the results, but these are complicated and expensive procedures.

An object of the present invention is thus to provide a simpler method for determining HIV-1 subtypes.

Another object of the present invention is to provide a kit for determining HIV-1 subtypes.

### BRIEF DESCRIPTION OF THE DRAWINGS

Figure 1 illustrates nucleotide sequences of the 5' adjacent region (C2 region) of the V3 region for the env gene in various subtypes of HIV-1. Capital letters indicate nucleotides that are entirely the same within a given subtype, and lower case letters indicate nucleotide variants within a given subtype. A question mark indicates that a consensus nucleotide was not determined because of too many variants. A dash indicates a nucleotide identical to that in subtype A. A period indicates the absence of a nucleotide in the corresponding site.
Figure 2 illustrates nucleotide sequences of the 3' adjacent region (C3 region) of the V3 region for the env gene in various subtypes of HIV-1. Capital letters indicate nucleotides that are entirely the same within a given subtype, and lower case letters indicate nucleotide variants within a given subtype. A question mark indicates that a consensus nucleotide was not determined because of too many variants. A dash indicates a nucleotide identical to that in subtype A. A period indicates the absence of a nucleotide in the corresponding site.
Figure 3 illustrates the locations, combinations, and base sequences of primers used in nested PCR (different primer pairs used for first and second PCR) for determining HIV-1 subtypes.
Figure 4 gives the results obtained when subtypes were detected by nested PCR using the primers illustrated in Figure 3 for samples in which the subtypes had been determined by sequencing of the virus genome.
Figure 5: Location of primers in HIV-1 subtype-specific nested PCR

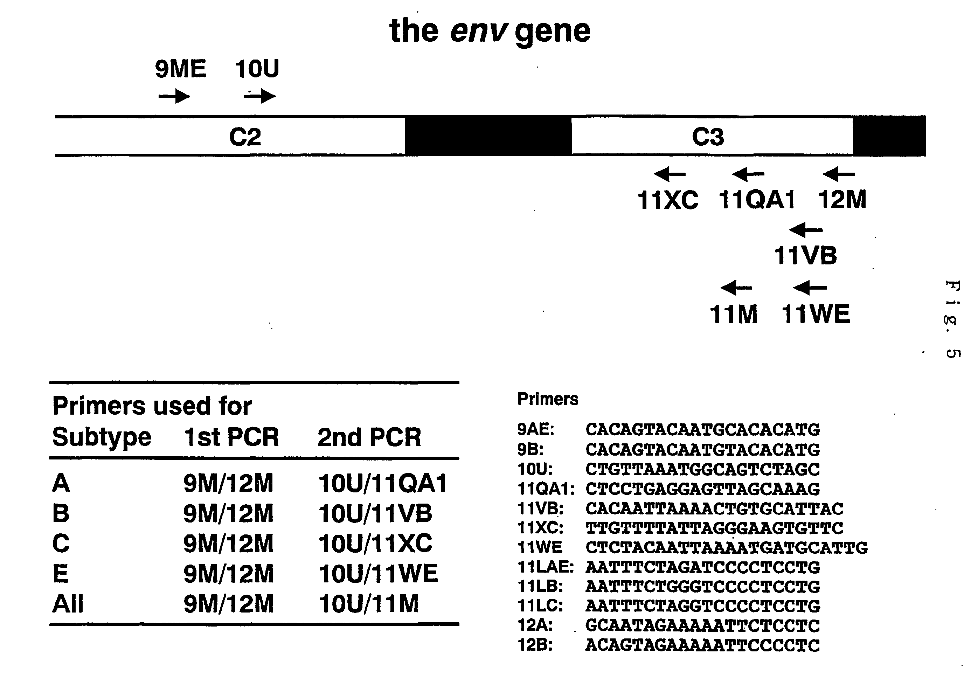

Figure 5 illustrates the locations, combinations, and base sequences of primers used in nested PCR for determining HIV-1 subtypes. 9M indicates a mixture of primers 9AE and 9B; 11M indicates a mixture of primers 11LAE, 11LB, and 11LC; and 12M indicates a mixture of primers 12A and 12B.

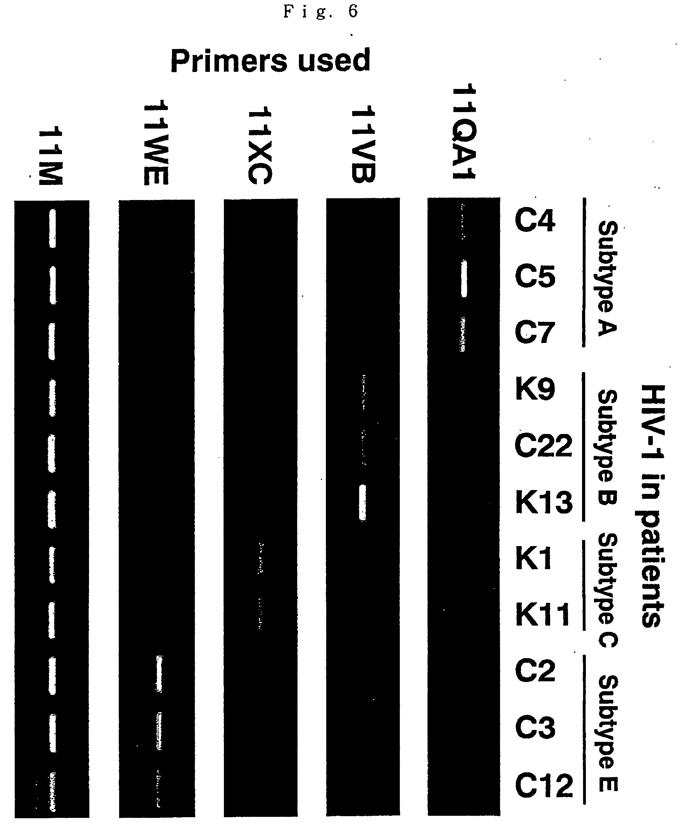

Figure 6: Subtype-specific PCR of HIV-1 DNA.
Figure 6 gives the results obtained when subtypes were detected by nested PCR using the primers illustrated in Figure 5.

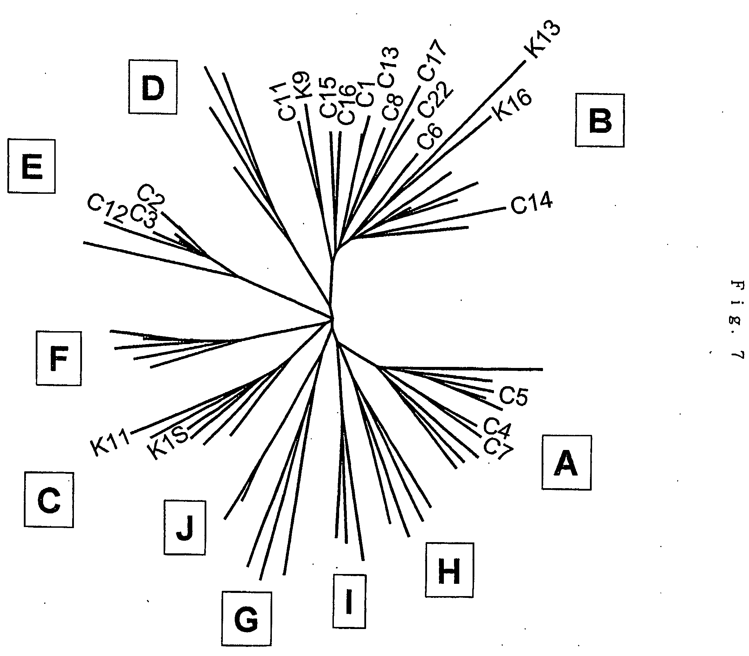

Figure 7: Phylogenetic analysis of HIV-1 variants
Figure 7 gives the results of subtypes obtained by phylogenetic analysis of HIV-1 variants based on the base sequence of the V3 region of the env gene obtained through sequencing.

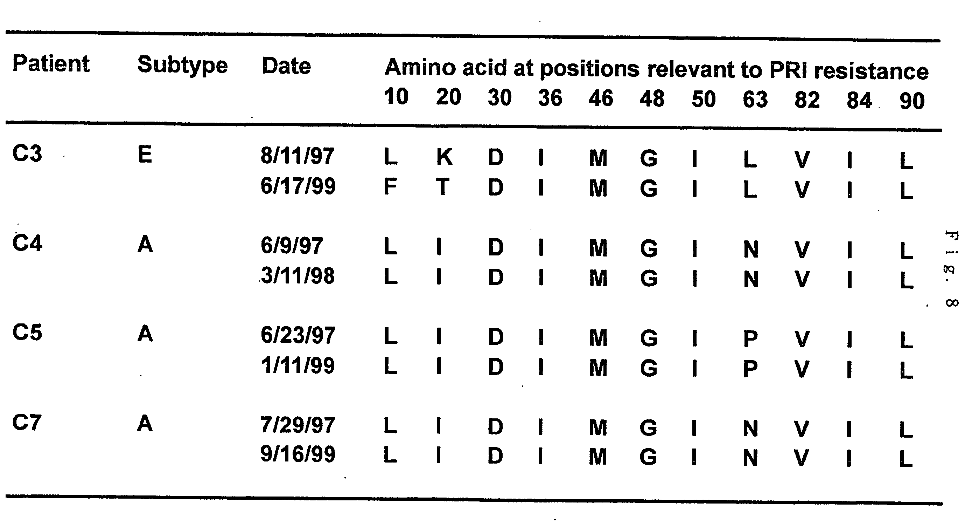

Figure 8: Amino acid sequence in PR of non-subtype B HIV-1 in patients receiving HAART
Figure 8 illustrates the amino acid sequences related to protease inhibitor resistance in non-subtype B HIV-1 patients receiving HAART.

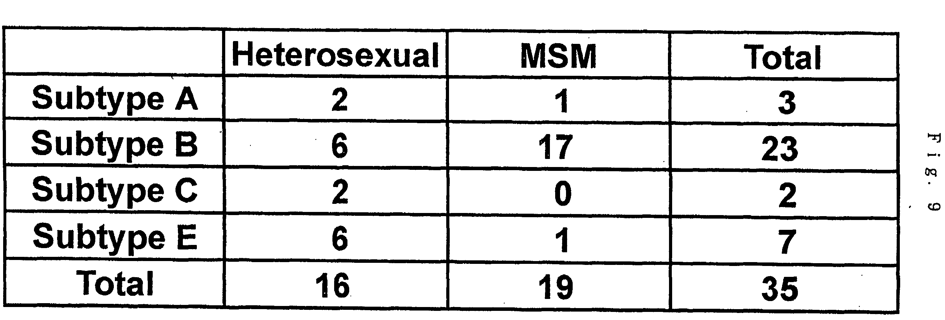

Figure 9 is a table of the correlation between various subtypes and the sexual behavior of HIV-1 patients.

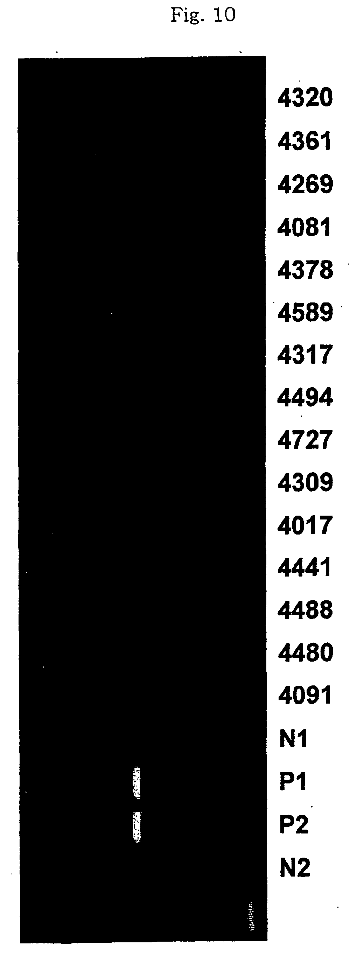

Figure 10: RT-PCR of RNA from PA⁺ but WB⁻ plasma with universal primers
Figure 10 gives the results obtained in RT-PCR using primer pairs allowing HIV-1 to be amplified irrespective of subtype in samples of serum from patients diagnosed as particle adsorption-positive (PA⁺) but Western blotting negative (WB⁻). N1 and N2 are negative controls, while P1 and P2 are positive controls.

### DISCLOSURE OF THE INVENTION

The inventors have designed various subtype-specific primers and have successfully used them to amplify nucleic acid in samples for rapid determination of HIV-1 subtypes, thereby perfecting the present invention.

Specifically, the present invention provides a method for determining HIV-1 subtypes, characterized by comprising the steps of amplifying nucleic acid using as a target sequence a portion of a nucleotide sequence of the env gene of HIV-1, where at least one of the 5' terminal and 3' terminal nucleotide sequences is different depending on the HIV-1 subtype, and detecting the subtype depending on whether or not the nucleic acid has been amplified. The target sequence should be 100 to 2500 base pairs in length, and preferably 150 to 500 base pairs in length. In the above method, the sequence from the 1^{st} through 30^{th} bases from the 3' terminal and/or 5' terminal of the target sequence should be different depending on the subtype. For example, the 3' terminal of the target sequence may be the C3 region of the env gene of HIV-1. The 5' terminal of the target sequence may be the C2 region of the env gene of HIV-1. Different subtypes can be detected by different amplification reactions using different primer pairs. For example, at least two subtypes can be distinguished following amplification carried out at least twice with two different pairs of primers of which 3' primers (primers 1) include sequences complementary to portions of the nucleotide sequence (nucleotide sequence 1) that differs depending on subtype in the C3 region of the env gene of HIV-1, and 5' primers (primers 2) include sequences complementary to portions of the nucleotide sequence (nucleotide sequence 2) of the C2 region of the env gene of HIV-1.

A first amplification reaction may be carried out with a first pair of primers using as a target sequence a portion of a nucleotide sequence of the env gene of HIV-1, a second amplification reaction may then be carried out with a second pair of primers using as a target sequence a portion of the aforementioned nucleotide sequence, where at least one of the 5' terminal and 3' terminal nucleotide sequences is different depending on the HIV-1 subtype, and the subtype may be detected depending on whether or not the nucleic acid has been amplified by the second amplification reaction. For example, the second pair of primers may consist of a primer (primer 1) that includes a sequence complementary to a portion of the nucleotide sequence (nucleotide sequence 1) that differs depending on subtype in the C3 region of the env gene of HIV-1, and a primer (primer 2) that includes a sequence complementary to a portion of the nucleotide sequence (nucleotide sequence 2) of the C2 region of the env gene of HIV-1; and the first pair of primers may consist of a primer (primer 3) that includes a sequence complementary to a portion of a nucleotide sequence (nucleotide sequence 3) of a region downstream of the 3' terminal of nucleotide sequence 1 of the env gene of HIV-1, and a primer (primer 4) that includes a sequence complementary to a portion of the nucleotide sequence (nucleotide sequence 4) of a region upstream of the 5' terminal of nucleotide sequence 2 of the env gene of HIV-1.

At least two subtypes can also be distinguished by repeating at least once the following series of operations with different pairs of second primers, where the operations comprise a first amplification reaction that is carried out with the first pair of primers using as a target sequence a portion of a nucleotide sequence of the env gene of HIV-1, a second amplification reaction that is then carried out with the second pair of primers using as a target sequence a nucleotide sequence within the above target sequence, and the detection of subtypes depending on whether or not the nucleic acid has been amplified by the second amplification reaction. For example, subtypes A, B, C, and E can be distinguished by: (a) detecting subtype A using as the first primer pair a mixture of primer 12A containing nucleotide sequence GCAATAGAAAAATTCTCCTC (Sequence ID No. 5) and primer 12B containing nucleotide sequence ACAGTAGAAAAATTCCCCTC (Sequence ID No. 6), and a mixture of primer 9AE containing nucleotide sequence CACAGTACAATGCACACATG (Sequence ID No. 8) and primer 9B containing nucleotide sequence CACAGTACAATGTACACATG (Sequence ID No. 9), and using as the second primer pair primer 11QA1 containing nucleotide sequence CTCCTGAGGAGTTAGCAAAG (Sequence ID No. 27) and primer 10U containing nucleotide sequence CTGTTAAATGGCAGTCTAGC (Sequence ID No. 20);
(b) detecting subtype B using as the first primer pair a mixture of primer 12A containing nucleotide sequence GCAATAGAAAAATTCTCCTC (Sequence ID No. 5) and primer 12B containing nucleotide sequence ACAGTAGAAAAATTCCCCTC (Sequence ID No. 6), and a mixture of primer 9AE containing nucleotide sequence CACAGTACAATGCACACATG (Sequence ID No. 8) and primer 9B containing nucleotide sequence CACAGTACAATGTACACATG (Sequence ID No. 9), and using as the second primer pair primer 11VB containing nucleotide sequence CACAATTAAAACTGTGCATTAC (Sequence ID No. 28) and primer 10U containing nucleotide sequence CTGTTAAATGGCAGTCTAGC (Sequence ID No. 20);
(c) detecting subtype C using as the first primer pair a mixture of primer 12A containing nucleotide sequence GCAATAGAAAAATTCTCCTC (Sequence ID No. 5) and primer 12B containing nucleotide sequence ACAGTAGAAAAATTCCCCTC (Sequence ID No. 6), and a mixture of primer 9AE containing nucleotide sequence CACAGTACAATGCACACATG (Sequence ID No. 8) and primer 9B containing nucleotide sequence CACAGTACAATGTACACATG (Sequence ID No. 9), and using as the second primer pair primer 11XC containing nucleotide sequence TTGTTTTATTAGGGAAGTGTTC (Sequence ID No. 29) and primer 10U containing nucleotide sequence CTGTTAAATGGTAGTCTAGC (Sequence ID No. 24); and
(d) detecting subtype C using as the first primer pair a mixture of primer 12A containing nucleotide sequence GCAATAGAAAAATTCTCCTC (Sequence ID No. 5) and primer 12B containing nucleotide sequence ACAGTAGAAAAATTCCCCTC (Sequence ID No. 6), and a mixture of primer 9AE containing nucleotide sequence CACAGTACAATGCACACATG (Sequence ID No. 8) and primer 9B containing nucleotide sequence CACAGTACAATGTACACATG (Sequence ID No. 9), and using as the second primer pair primer 11WE containing nucleotide sequence CTCTACAATTAAAATGATGCATTG (Sequence ID No. 30) and primer 10U containing nucleotide sequence CTGTTAAATGGCAGTCTAGC (Sequence ID No. 20).

Alternatively, at least two subtypes can be distinguished by repeating at least once the following series of operations with different pairs of first and second primers, where the operations comprise a first amplification reaction that is carried out with a first pair of primers using as a target sequence a portion of a nucleotide sequence of the env gene of HIV-1, a second amplification reaction that is then carried out with a second pair of primers using as a target sequence a nucleotide sequence within the target sequence in the first reaction, and the detection of subtypes depending on whether or not the nucleic acid has been amplified by the second amplification reaction. For example, subtypes A, B, and E can be distinguished by: (a) detecting subtype A using as the first primer pair primer 12A containing nucleotide sequence GCAATAGAAAAATTCTCCTC (Sequence ID No. 5) and primer 9AE containing nucleotide sequence CACAGTACAATGCACACATG (Sequence ID No. 8), and using as the second primer pair primer 11QA containing nucleotide sequence CTCCTGAGGGGTTAGCAAAG (Sequence ID No. 1) and primer 10 containing nucleotide sequence AAATGGCAGTCTAGCAGAAG (Sequence ID No. 4);
(b) detecting subtype B using as the first primer pair primer 12B containing nucleotide sequence ACAGTAGAAAAATTCCCCTC (Sequence ID No. 6) and primer 9B containing nucleotide sequence CACAGTACAATGTACACATG (Sequence ID No. 9), and using as the second primer pair primer 11BB containing nucleotide sequence CTGTGCATTACAATTTCTGG (Sequence ID No. 2) and primer 10 containing nucleotide sequence AAATGGCAGTCTAGCAGAAG (Sequence ID No. 4); and
(c) detecting subtype E using as the first primer pair primer 12E containing nucleotide sequence GCAATAGAAAAATTCCCCTC (Sequence ID No. 7) and primer 9AE containing nucleotide sequence CACAGTACAATGCACACATG (Sequence ID No. 8), and using as the second primer pair primer 11QE containing nucleotide sequence CTCCTGAGGGTGGTTGAAAG (Sequence ID No. 3) and primer 10 containing nucleotide sequence AAATGGCAGTCTAGCAGAAG (Sequence ID No. 4).

The method of the present invention may further comprise the steps of amplifying nucleic acid using as a target sequence a portion of a nucleotide sequence of the HIV-1 genome, the nucleotide sequence being highly conserved among all subtypes, and ascertaining the presence or absence of HIV-1 depending on whether or not the nucleic acid has been amplified. The step for ascertaining the presence or absence of HIV-1 comprises amplifying the nucleic acid with a first primer pair using as a target sequence a portion of a nucleotide sequence of the HIV-1 genome, the nucleotide sequence being highly conserved among all subtypes, then carrying out a second amplifying reaction with a second primer pair using as a target sequence a nucleotide sequence in the above target sequence, and ascertaining the presence or absence of HIV-1 depending on whether or not the nucleic acid has been amplified. The first primers referred to here may comprise a mixture of primer 12A containing nucleotide sequence GCAATAGAAAAATTCTCCTC (Sequence ID No. 5), primer 12B containing nucleotide sequence ACAGTAGAAAAATTCCCCTC (Sequence ID No. 6), primer 9AE containing nucleotide sequence CACAGTACAATGCACACATG (Sequence ID No. 8), and primer 9B nucleotide sequence CACAGTACAATGTACACATG (Sequence ID No. 9), and the second primer pair may comprise primer 11LB containing nucleotide sequence AATTTCTGGGTCCCCTCCTG (Sequence ID No. 18), primer 11LAE containing nucleotide sequence AATTTCTAGATCCCCTCCTG (Sequence ID No. 25), primer 11LC containing nucleotide sequence AATTTCTAGGTCCCCTCCTG (Sequence ID No. 26), and primer 10U containing nucleotide sequence CTGTTAAATGGCAGTCTAGC (Sequence ID No. 20).

Another object of the present invention is to provide a kit for determining HIV-1 subtypes, comprising primer pairs in which a target sequence is a portion of a nucleotide sequence of the env gene of HIV-1, where at least one of the 5' terminal and 3' terminal nucleotide sequences is different depending on the subtype.

Embodiments of the present invention are illustrated below.

A sample of blood, lymph, spinal fluid, semen, lymph node, or the like is taken from individuals suspected of HIV-1 infection, infected individuals and patients confirmed with HIV-1 infection, patients being treated for HIV-1, and the like. DNA is extracted using a QIAamp Blood Kit by QIAGEN, either directly or after monocytes have been isolated from the sample by Ficoll-Paque gradient centrifugation (Pharmacia). Alternatively, RNA is extracted using a QIAamp Viral RNA Kit by QIAGEN from plasma. The DNA or RNA concentration is then determined base on the optical resolution at 260 nm.

The nucleic acid is then treated in PCR, and preferably nested PCR.

The use of nested PCR is described below. Nested PCR involves designing a second primer pair inside a target sequence amplified with another primer pair (first primer pair), carrying out a first PCR step, and then carrying out a second PCR step, and then diluting the reaction product as new template for a second PCR step. Undesirable sequences are sometimes amplified in addition to the target sequence in the first PCR step. However, there is very little probability that undesirable fragments amplified during the first PCR step will have a sequence with which the primers of the second primer pair will anneal. The second PCR step is thus carried out for selective amplification of only the target sequence.

The initial PCR step (first PCR) is first carried out using different primers for each subtype to be distinguished (such as subtype A, subtype B, and subtype E). Alternatively, universal primer pairs allowing any type of subtype to be amplified can be used instead of subtype-specific primer pairs.

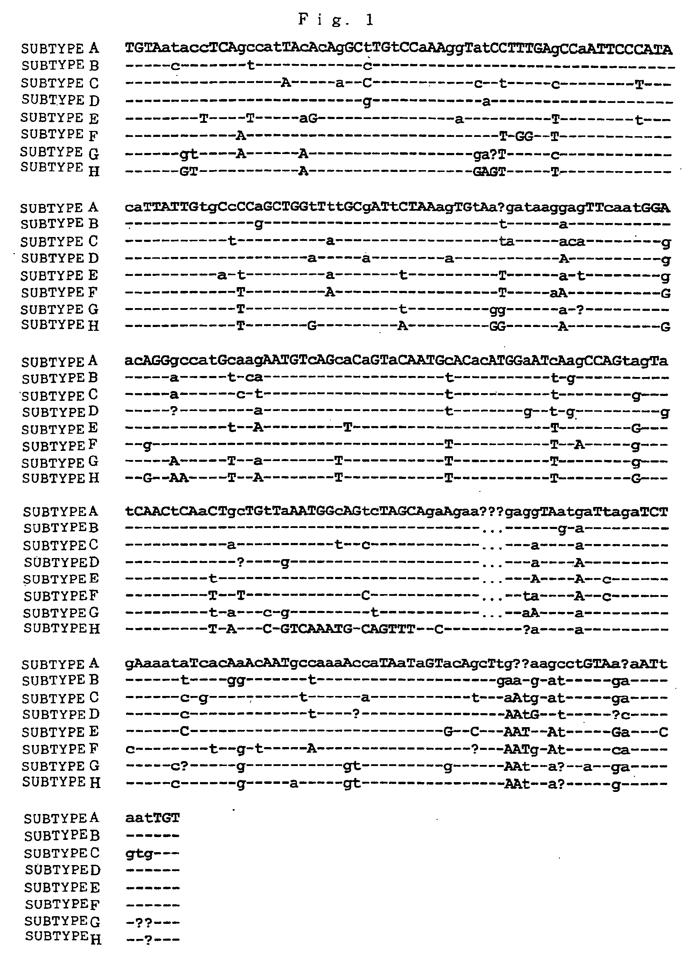

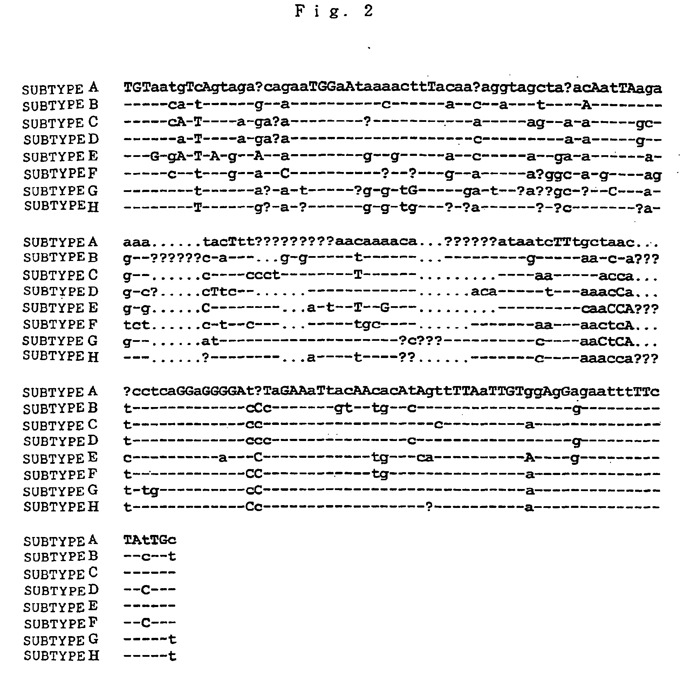

An example of a subtype-specific primer pair is a primer pair consisting of a primer (primer 4') which includes a sequence complementary to a portion of a nucleotide sequence in the C2 region of the env gene of HIV-1 and a primer (primer 3') which includes a sequence complementary to a portion of the nucleotide sequence of the C3 region of the env gene for HIV-1 that differs depending on subtype (that is, subtype-specific nucleotide sequence). Since the C2 region of the env gene of HIV-1 has a nucleotide sequence that differs depending on the subtype, as shown in Figure 1, the nucleotide sequence may be selected from this region to design primer 4'. Because the C3 region of the env gene of HIV-1 varies depending on the subtype, as shown in Figure 2, the nucleotide sequence may be selected from this region to design primer 3'. The primer length should generally be 18 to 30 base pairs, and preferably 20 to 25 base pairs. The following primers can be specifically used.

Primer pairs and their nucleotide sequences for first PCR to detect subtype A

9AE/12A
primer 9AE: CACAGTACAATGCACACATG (Sequence ID No. 8)
primer 12A: GCAATAGAAAAATTCTCCTC (Sequence ID No. 5)

Primer 9AE is a subtype A, E, F, and H-specific primer in which the sequence is complementary to the sequence from 6943 to 6962, counting from the 5' terminal (left terminal), of the complete base sequence for HIV-1 (NL4-3 strain).

Primer 12A is a subtype A, C, E, G, H, I, and J-specific primer in which the sequence is complementary to the sequence from 7369 to 7350, counting from the 5' terminal (left terminal), of the complete base sequence for HIV-1 (NL4-3 strain).

Primer pairs and their nucleotide sequences for first PCR to detect subtype B

9B/12B
primer 9B: CACAGTACAATGTACACATG (Sequence ID No. 9)
primer 12B: ACAGTAGAAAAATTCCCCTC (Sequence ID No. 6)

Primer 9B is a subtype B, C, D, E, F, G, H, and J-specific primer in which the sequence is complementary to the sequence from 6943 to 6962, counting from the 5' terminal (left terminal), of the complete base sequence for HIV-1 (NL4-3 strain).

Primer 12B is a subtype B, D, E, F, and I-specific primer in which the sequence is complementary to the sequence from 7369 to 7350, counting from the 5' terminal (left terminal), of the complete base sequence for HIV-1 (NL4-3 strain).

Primer pairs and their nucleotide sequences for first PCR to detect subtype E

9AE/12E
primer 9AE: CACAGTACAATGCACACATG (Sequence ID No. 8)
primer 12E: GCAATAGAAAAATTCCCCTC (Sequence ID No. 7)

Primer 12E is a primer specific to subtype E only, in which the sequence is complementary to the sequence from 6943 to 6962, counting from the 5' terminal (left terminal), of the complete base sequence for HIV-1 (NL4-3 strain).

Primer pairs and their nucleotide sequences for first PCR to detect subtype C

9B/12A
primer 9B: CACAGTACAATGTACACATG (Sequence ID No. 9)
primer 12A: GCAATAGAAAAATTCTCCTC (Sequence ID No. 5)

Primer pairs and their nucleotide sequences for first PCR to detect subtype D

9B/12B
primer 9B: CACAGTACAATGTACACATG (Sequence ID No. 9)
primer 12B: ACAGTAGAAAAATTCCCCTC (Sequence ID No. 6)

Primer pairs and their nucleotide sequences for first PCR to detect subtype F

9B/12A
primer 9B: CACAGTACAATGTACACATG (Sequence ID No. 9)
primer 12A: GCAATAGAAAAATTCTCCTC (Sequence ID No. 5)

Primer pairs and their nucleotide sequences for first PCR to detect subtype G

9B/12A
primer 9B: CACAGTACAATGTACACATG (Sequence ID No. 9)
primer 12A: GCAATAGAAAAATTCTCCTC (Sequence ID No. 5)

Primer pairs and their nucleotide sequences for first PCR to detect subtype H

9B/12A
primer 9B: CACAGTACAATGTACACATG (Sequence ID No. 9)
primer 12A: GCAATAGAAAAATTCTCCTC (Sequence ID No. 5)

First PCR may alternatively be carried out using a primer mixture capable of giving amplified products for several subtypes. An example of a primer for such a purpose is a mixture of primers 9AE, primer 9B, primer 12A, and primer 12B.

1/1000 to 1/5 (for example, 1/50) of the PCR products is used to carry out the next PCR (second PCR) with another pair of primers that differ by subtype. The pair of primers for second PCR is designed from within the target sequence amplified during the first PCR. For example, at least one of the primers forming the subtype-specific primer pair for second PCR can be a primer (primer 1) containing a sequence complementary to a portion of the subtype-specific nucleotide sequence of the C2 region of the env gene for HIV-1. Because the nucleotide sequence of the C2 region of the env gene for HIV-1 differs by subtype, as shown in Figure 1, a nucleotide sequence from this region can be selected to design the primer. Figure 2 gives the nucleotide sequence of the 3' adjacent region (C3 region) of the V3 region of the env gene for various subtypes of HIV-1. Since the nucleotide sequence varies depending on the subtype, a suitable sequence can be selected to design a primer. To design a subtype-specific primer, phylogenetic analysis is employed to select nucleotide sequences of a given subtype which are as genetically remote as possible from the corresponding nucleotide sequence of other subtypes. An example can include a primer (primer 2) containing a sequence complementary to a portion of the nucleotide sequence of the C3 region of the env gene for HIV-1. The primers pairs containing the following nucleotide sequences are specific examples.

Primer pairs and their nucleotide sequences for second PCR to detect subtype A

10/11QA
primer 10: AAATGGCAGTCTAGCAGAAG (Sequence ID No. 4)
primer 11QA: CTCCTGAGGGGTTAGCAAAG (Sequence ID
No. 1)

Primer 10 is a subtype A, B, D, and E-specific primer in which the sequence is complementary to the sequence from 6997 to 7016, counting from the 5' terminal (left terminal), of the complete base sequence for HIV-1 (NL4-3 strain).

Primer 11QA is a primer specific to only subtype A, in which the sequence is complementary to the sequence from 7313 to 7294, counting from the 5' terminal (left terminal), of the complete base sequence for HIV-1 (NL4-3 strain).

10U/11QA1
primer 10U: CTGTTAAATGGCAGTCTAGC (Sequence ID No. 20)
primer 11QA1: CTCCTGAGGAGTTAGCAAAG (Sequence ID No. 27)

Primer 10U is a subtype A, B, D, E, and J-specific primer in which the sequence is complementary to the sequence from 6992 to 7011, counting from the 5' terminal (left terminal), of the complete base sequence for HIV-1 (NL4-3 strain).

Primer 11QA1 is a primer specific to only subtype A, in which the sequence is complementary to the sequence from 7313 to 7294, counting from the 5' terminal (left terminal), of the complete base sequence for HIV-1 (NL4-3 strain).

Primer pairs and their nucleotide sequences for second PCR to detect subtype B

10/11BB
primer 10: AAATGGCAGTCTAGCAGAAG (Sequence ID No. 4)
primer 11BB: CTGTGCATTACAATTTCTGG (Sequence ID No. 2)

Primer 11BB is a primer specific to only subtype B, in which the sequence is complementary to the sequence from 7338 to 7319, counting from the 5' terminal (left terminal), of the complete base sequence for HIV-1 (NL4-3 strain).

10U/11VB
primer 10U: CTGTTAAATGGCAGTCTAGC (Sequence ID No. 20)
primer 11VB: CACAATTAAAACTGTGCATTAC (Sequence ID No. 28)

Primer 11VB is a primer specific to only subtype B, in which the sequence is complementary to the sequence from 7349 to 7328, counting from the 5' terminal (left terminal), of the complete base sequence for HIV-1 (NL4-3 strain).

Primer pairs and their nucleotide sequences for second PCR to detect subtype E

10/11QE
primer 10: AAATGGCAGTCTAGCAGAAG (Sequence ID No. 4)
primer 11QE: CTCCTGAGGGTGGTTGAAAG (Sequence ID No. 3)

Primer 11QE is a primer specific to only subtype E, in which the sequence is complementary to the sequence from 7313 to 7294, counting from the 5' terminal (left terminal), of the complete base sequence for HIV-1 (NL4-3 strain).

10U/11WE

primer 10U: CTGTTAAATGGCAGTCTAGC (Sequence ID No. 20)

primer 11WE: CTCTACAATTAAAATGATGCATTG (Sequence ID No. 30)

Primer 11WE is a primer specific to only subtype E, in which the sequence is complementary to the sequence from 7352 to 73339, counting from the 5' terminal (left terminal), of the complete base sequence for HIV-1 (NL4-3 strain).

Primer pairs and their nucleotide sequences for second PCR to detect subtype C

10C/11RC
primer 10C: AAATGGTAGCCTAGCAGAAG (Sequence ID No. 10)
primer 11RC: CTCCTGAGGATGGTGCAAATTT (Sequence ID No. 13)

Primer 10C is a subtype C and F-specific primer in which the sequence is complementary to the sequence from 6997 to 7016, counting from the 5' terminal (left terminal), of the complete base sequence for HIV-1 (NL4-3 strain).

Primer 11RC is a primer specific to only subtype C, in which the sequence is complementary to the sequence from 7313 to 7292, counting from the 5' terminal (left terminal), of the complete base sequence for HIV-1 (NL4-3 strain).

10U/11XC
primer 10U: AAATGGTAGCCTAGCAGAAG (Sequence ID No. 10)
primer 11XC: TTGTTTTATTAGGGAAGTGTTC (Sequence ID No. 29)

Primer 11XC is a primer specific to only subtype C, in which the sequence is complementary to the sequence from 7289 to 7268, counting from the 5' terminal (left terminal), of the complete base sequence for HIV-1 (NL4-3 strain).

Primer pairs and their nucleotide sequences for second PCR to detect subtype D

10/11RD
primer 10: AAATGGCAGTCTAGCAGAAG (Sequence ID No. 4)
primer 11RD: CTCCTGAGGATGGTTTAAAAAT (Sequence ID No. 14)

Primer 11RD is a primer specific to only subtype D, in which the sequence is complementary to the sequence from 7313 to 7292, counting from the 5' terminal (left terminal), of the complete base sequence for HIV-1 (NL4-3 strain).

Primer pairs and their nucleotide sequences for second PCR to detect subtype F

10C/11RF
primer 10C: AAATGGTAGCCTAGCAGAAG (Sequence ID No. 10)
primer 11RF: CTCCTGAGGATGAGTTAAATTT (Sequence ID No. 15)

Primer 11RF is a primer specific to only subtype F, in which the sequence is complementary to the sequence from 7313 to 7292, counting from the 5' terminal (left terminal), of the complete base sequence for HIV-1 (NL4-3 strain).

Primer pairs and their nucleotide sequences for second PCR to detect subtype G

10G/11SG
primer 10G: GAATGGCAGTTTAGCAGAAG (Sequence ID No. 11)
primer 11SG: TCCTGCAGATGAGTTAAAGG (Sequence ID No. 16)

Primer 10G is a primer specific to only subtype G, in which the sequence is complementary to the sequence from 6997 to 7016, counting from the 5' terminal (left terminal), of the complete base sequence for HIV-1 (NL4-3 strain).

Primer 11SG is a primer specific to only subtype G, in which the sequence is complementary to the sequence from 7312 to 7293, counting from the 5' terminal (left terminal), of the complete base sequence for HIV-1 (NL4-3 strain).

Primer pairs and their nucleotide sequences for second PCR to detect subtype H

10H/11SH
primer 10H: GTCAAATGGCAGTTTAGCAG (Sequence ID No. 12)
primer 11SH: TCCTGAGGATGGTTTAAAGG (Sequence ID No. 17)

Primer 10H is a primer specific to only subtype H, in which the sequence is complementary to the sequence from 6994 to 7013, counting from the 5' terminal (left terminal), of the complete base sequence for HIV-1 (NL4-3 strain).

Primer 11SH is a primer specific to only subtype H, in which the sequence is complementary to the sequence from 7312 to 7293, counting from the 5' terminal (left terminal), of the complete base sequence for HIV-1 (NL4-3 strain).

Second PCR may alternatively be carried out using a mixture of primers capable of giving amplified products for several subtypes in order to permit the amplification of any subtype. Examples of primers for that purpose include the following primer mixtures.

primer 10U: AAATGGCAGTCTAGCAGAAG (Sequence ID No. 4)

primer 11LB: AATTTCTGGGTCCCCTCCTG (Sequence ID No. 18)

primer 11LAE: AATTTCTAGATCCCCTCCTG (Sequence ID No. 25)

primer 11LC: AATTTCTAGGTCCCCTCCTG (Sequence ID No. 26)

Primer 11LB is a subtype B, D, F, G, and I-specific primer in which the sequence is complementary to the sequence from 7327 to 7308, counting from the 5' terminal (left terminal), of the complete base sequence for HIV-1 (NL4-3 strain).

Primer 11LAE is a subtype A, E, F, G, I, and J-specific primer in which the sequence is complementary to the sequence from 7327 to 7308, counting from the 5' terminal (left terminal), of the complete base sequence for HIV-1 (NL4-3 strain).

Primer 11LC is a subtype C, F, G, H, I, and J-specific primer in which the sequence is complementary to the sequence from 7327 to 7308, counting from the 5' terminal (left terminal), of the complete base sequence for HIV-1 (NL4-3 strain).

The following primers can also be used.

Primer 10KC: CTCAACTACTGTTAAATGGTAG (Sequence ID No. 21)

Primer 10KC is a primer specific to only subtype C, in which the sequence is complementary to the sequence from 6984 to 7005, counting from the 5' terminal (left terminal), of the complete base sequence for HIV-1 (NL4-3 strain).

Primer 10UF: CTGTTAAATGGCAGCCTAGC (Sequence ID No. 22)

Primer 10UF is a subtype A, E, F, H, and I-specific primer in which the sequence is complementary to the sequence from 6992 to 7011, counting from the 5' terminal (left terminal), of the complete base sequence for HIV-1 (NL4-3 strain).

Primer 10UG: CTGTTAAATGGCAGTTTAGC (Sequence ID No. 23)

Primer 10UG is a subtype A, E, G, I, and J-specific primer in which the sequence is complementary to the sequence from 6992 to 7011, counting from the 5' terminal (left terminal), of the complete base sequence for HIV-1 (NL4-3 strain).

Primer 10UC: CTGTTAAATGGTAGTCTAGC (Sequence ID No. 24)

Primer 10UC is a subtype C and E-specific primer in which the sequence is complementary to the sequence from 6992 to 7011, counting from the 5' terminal (left terminal), of the complete base sequence for HIV-1 (NL4-3 strain).

Primer 11LE: AATTTCTAGATCTCCTCCTG )Sequence ID No. 19)

Primer 11LE is a subtype E, F, G, H, and J-specific primer in which the sequence is complementary to the sequence from 7327 to 7308, counting from the 5' terminal (left terminal), of the complete base sequence for HIV-1 (NL4-3 strain).

Primer 11LC: AATTTCTAGGTCCCCTCCTG (Sequence ID No. 26)

Primer 11LC is a subtype C, F, G, H, I, and J-specific primer in which the sequence is complementary to the sequence from 7327 to 7308, counting from the 5' terminal (left terminal), of the complete base sequence for HIV-1 (NL4-3 strain).

Primer 11TC: TTCTCCTCTACAATTAAAGC (Sequence ID No. 31)

Primer 11TC is a primer specific to only subtype C, in which the sequence is complementary to the sequence from 7357 to 7338, counting from the 5' terminal (left terminal), of the complete base sequence for HIV-1 (NL4-3 strain).

Primer 11RC1: TTATTGTTTTATTAGGGAAGTG (Sequence ID No. 32)

Primer 11RC1 is a primer specific to only subtype C, in which the sequence is complementary to the sequence from 7292 to 7271, counting from the 5' terminal (left terminal), of the complete base sequence for HIV-1 (NL4-3 strain).

Primer 11SE: TGCATTGTAATTTCTAGATCTC (Sequence ID No. 33)

Primer 11SE is a primer specific to only subtype E, in which the sequence is complementary to the sequence from 7333 to 7314, counting from the 5' terminal (left terminal), of the complete base sequence for HIV-1 (NL4-3 strain).

Primer 11BE: TGATGCATTGTAATTTCTAG (Sequence ID No. 34)

Primer 11BE is a primer specific to only subtype E, in which the sequence is complementary to the sequence from 7338 to 7319, counting from the 5' terminal (left terminal), of the complete base sequence for HIV-1 (NL4-3 strain).

The PCR procedures and reaction conditions may be in accordance with those in Bruisten S. et al., *AIDS Res Hum Retroviruses* 1993, 9:259-265, but the hot start method is preferred. In hot start PCR, the PCR reaction solution is kept on a hot plate for start up at an elevated temperature (usually 90°C or more ).

However, it sometimes happens that no subtype is detected in attempts to determine the HIV-1 subtype in such a method. Possible causes may be that the HIV-1 DNA concentration is below the detection threshold, or the presence of numerous variants at the primer binding site.

To deal with the former possibility, the above method can be implemented after the extraction of the RNA from plasma, since the concentration of HIV-1 is generally higher in plasma than in cells, and its subsequent conversion to DNA using reverse transcriptase.

To deal with the latter possibility, the determination of the subtype by this method is held off, another genetic region of HIV-1 is amplified by PCR to determine the nucleotide sequence, and the subtype is determined by a conventional method (Note: HIV-1 infection is generally diagnosed by detecting antibodies. This invention is not a method for diagnosing HIV-1 infection.).

The PCR reaction products are separated by agarose gel electrophoresis and detected by ethidium bromide staining. Although distinct bands can be observed with the use of primers consistent with the subtype of the HIV-1 in sample DNA, the bands are indistinct or not observed at all when the primers are not. The HIV-1 subtype is determined in this way.

Determining the HIV-1 subtype in the present invention may include the steps of amplifying nucleic acid using as a target sequence a portion of a nucleotide sequence of the HIV-1 genome, where the nucleotide sequence is highly conserved among all subtypes, and determining the presence of absence of HIV-1 depending on whether or not the nucleic acid has been amplified. The step for ascertaining the presence or absence of HIV-1 may comprise amplifying the nucleic acid with a primer mixture for first PCR (such as 9AE/9B/12A/12B) using as a target sequence a portion of a nucleotide sequence of the HIV-1 genome, the nucleotide sequence being highly conserved among all subtypes, then carrying out a second amplifying reaction with a primer mixture for second PCR (such as 10U/11LB/11LAE/11LC) using as a target sequence a nucleotide sequence in the above target sequence, and then ascertaining the presence or absence of HIV-1 depending on whether or not the nucleic acid has been amplified.

The present invention also encompasses a kit for determining HIV-1 subtypes, comprising primer pairs in which a target sequence is a portion of a nucleotide sequence of the env gene of HIV-1, where at least one of the 5' terminal and 3' terminal nucleotide sequences is different depending on the subtype. Examples of primer pairs include primer pairs (inner primers) for second PCR such as those above, and combinations of primer pairs for first PCR (outer primers) and primer pairs for second PCR. The kit of the present invention may also include dNTP mixtures, reaction buffers, DNA polymerase, universal subtype primer pairs for first PCR, and universal subtype primer pairs for second PCR. To minimize the effects caused by inconsistencies between the primer and analyte HIV-1 DNA base pairs, the magnesium ion concentration of the reaction buffer should be increased from the usual concentration of 1.5 mM to 4 mM.

The components constituting the diagnostic kit may be packaged individually, assembled, or bundled in containers such as vials and tubes, and the containers may be bundled and housed in support means divided for housing such components.

### BEST MODE FOR CARRYING OUT THE INVENTION

The present invention is illustrated in detail in the following examples, but the scope of the present invention is not in any way limited by these examples.

### Example 1

### Subjects and Methodology

1) Subtype-specific reference specimens to study method for determining subtype
   Reference specimens were prepared after the extraction of DNA from the blood of 3, 8, and 3 HIV-1-infected patients; the subtypesof HIV-1 were determined to be A, B, and E, respectively, by env gene sequencing and phylogenetic analysis.
2) Subjects for determining subtype
   The HIV-1 subtype was determined in 8 subjects with HIV who either visited or were hospitalized in Tokyo hospitals.
3) Preparation of DNA from blood of HIV patients
   10 mL peripheral blood was drawn from the above HIV patients. Sodium citrate was used as an anticoagulant. Monocytes were separated from the peripheral blood by Ficoll-Paque (Pharmacia) gradient centrifugation, and DNA was then prepared using a QIAamp Blood Kit (QIAGEN). The DNA was dissolved in pure water or buffer containing 1 mM EDTA, and was stored at -20°C until use. 0.5 µg DNA was used in PCR.
4) Detection of subtypes A, B, and E by PCR

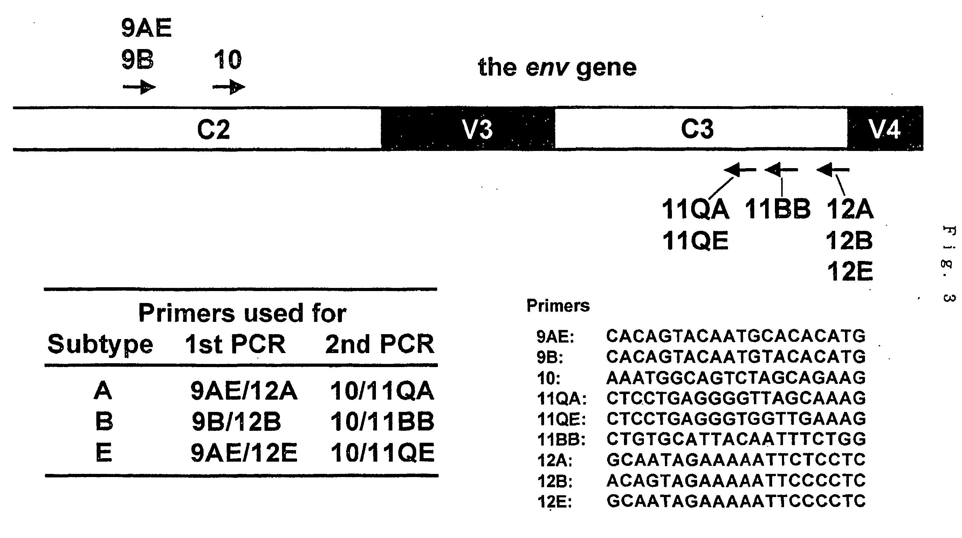

Figure 3 gives the nucleotide sequences of the primers used in PCR.

For subtype A-specific detection, nested PCR was carried out using 9AE and 12A as the primers for first PCR, and 10 and 11QA as the primers for second PCR. For subtype B-specific detection, nested PCR was carried out using 9B and 12B as the primers for first PCR, and 10 and 11BB as the primers for second PCR. For subtype E-specific detection, nested PCR was carried out using 9AE and 12E as the primers for first PCR, and 10 and 11QE as the primers for second PCR (Figure 3).

PCR was carried out for 30 cycles, where one cycle was 15 seconds at 94°C, 30 seconds at 56°C, and 1 minute at 72°C, with 100 µL reaction solution (10 mM Tris-HCl pH 8.3, 50 mM KCl, 4 mM MgCl₂, 0.2 mM dNTP, 1.0 µM primer, 2.5 units Taq polymerase) using 0.5 µg sample DNA prepared from HIV patients. Second PCR was carried out for 25 cycles under the same conditions using 2 µL reaction solution from the first PCR. 30 seconds at 60°C was used instead of 30 seconds at 56°C, however.

PCR products (subtypes A and E: 317 bp; subtype B: 342 bp) were detected by isolation through electrophoresis with 2% agarose gel, and by subsequent ethidium bromide staining.

### Results

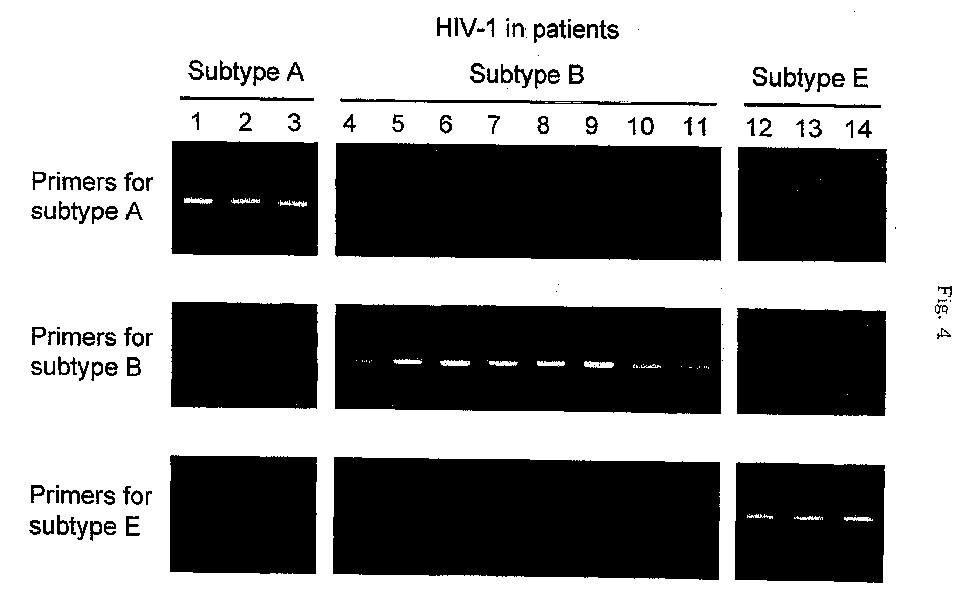

1) Study of subtype determination by PCR of subtype-specific reference samples
The following results were obtained for specimens whose subtype had been determined by virus genome sequencing. Only subtype A specimens were positive, while subtype B and E specimens were all negative, in PCR using primers for the detection of subtype A. Only subtype B specimens were positive, while subtype A and E specimens were all negative, in PCR using primers for the detection of subtype B. Only subtype E specimens were positive, while subtype A and B specimens were all negative, in PCR using primers for the detection of subtype E (Figure 4).
2) Determination of HIV patient subtype by PCR
Table 1 gives the results obtained in the determination of HIV-1 subtypes in 8 HIV patients who either visited or were hospitalized in Tokyo hospitals.

**Table 1:**

| results obtained in determination of subtypes in 8 specimens of unknown subtype | | | |
|---|---|---|---|
| Case | primer pair for subtype A | primer pair for subtype B | primer pair for subtype E |
| P18 | - | + | - |
| P19 | - | + | - |
| P20 | - | + | - |
| P21 | - | - | + |
| P22 | - | ? | - |
| P23 | - | - | + |
| P24 | - | + | - |
| P25 | - | + | - |

In the table above, + denotes detection of HIV-1 specific DNA bands, - denotes non-detection thereof. The symbol ? for case P22 denotes detection of shorter bands than prediction.

Based on these results, Cases P18, P19, P20, P24, and P25 were diagnosed as being infected with subtype B, and Cases P21 and P23 were diagnosed as being infected with subtype E. Although a DNA band was detected only with the use of a primer pair for subtype B in Case P22, it was shorter than expected, so determination was postponed. To verify that the above results were correct, the amplified DNA was sequenced and phylogenetically analyzed, giving results that were consistent with those in Table 1. Case P22 turned out to be subtype B.

The results in 1) and 2) demonstrate that this method was able to correctly diagnose the subtype in 21 out of 22 cases. The determination was postponed in the remaining one case. That is, the present method has been shown to be a simple and reliable method for determining subtypes.

The method of the present invention allows HIV-1 subtypes to be determined at a cost of about ¥2,000 per specimen. The time needed to determine the subtype in treating all 8 specimens at once was 2 hour for the isolation of the DNA, 6 hours for PCR, and about 1 hour for electrophoresis.

### Example 2

### Subjects and Methodology

1) Subtype-specific reference specimens for detection in subtype determination method
   Reference specimens were prepared from DNA extracted from the blood of 11 subjects, which included 2 patients with HIV-1 determined to be subtype C by env gene sequencing and phylogenetic analysis, in addition to the 3 subtype A HIV-1 subjects, 3 subtype B HIV-1 subjects, and 3 subtype E HIV-1 subjects used in Example 2.
2) Subjects for determining subtype
   The HIV-1 subtype was determined in 32 subjects with HIV who either visited or were hospitalized in Tokyo hospitals.
3) Preparation of DNA from blood of HIV patients
   10 mL peripheral blood was drawn from the above HIV patients. Sodium citrate was used as an anticoagulant. Monocytes were separated from the peripheral blood by Ficoll-Paque (Pharmacia) gradient centrifugation, and DNA was then prepared using a QIAamp Blood Kit (QIAGEN). The DNA was dissolved in pure water or buffer containing 1 mM EDTA, and was stored at -20°C until immediately before use. 0.5 µg DNA was used in PCR.
4) Detection of subtypes A, B, C, and E by PCR

Figure 5 gives the nucleotide sequences of the primers used in PCR.

A mixture of 9AE, 9B, 12A, and 12B was used for the primers in first PCR. Nested PCR was carried out using the following primers for second PCR: 10U and 11QA1 for subtype A-specific detection, 10U and 11VB for subtype B-specific detection, 10U and 11XC for subtype C-specific detection, and 10U and 11WE for subtype E-specific detection. Nested PCR was carried out using a mixture of 10U, 11LB, 11LAE, and 11LC for amplification of HIV-1 DNA irrespective of subtype (Figure 5).

PCR was carried out for 30 cycles, where one cycle was 15 seconds at 94°C, 30 seconds at 56°C, and 1 minute at 72°C, with 100 µL reaction solution (10 mM Tris-HCl pH 8.3, 50 mM KCl, 4 mM MgCl₂, 0.2 mM dNTP, 1.0 µM primer, 2.5 units Taq polymerase) using 0.5 µg sample DNA prepared from HIV patients. Second PCR was carried out for 25 cycles under the same conditions using 2 µL reaction solution from the first PCR. 30 seconds at 60°C was used instead of 30 seconds at 56°C, however.

PCR products (subtype A: 322 bp; subtype B: 358 bp; subtype C: 298 bp; subtype E: 361)) were detected by isolation through electrophoresis with 2% agarose gel, and by subsequent ethidium bromide staining.

### Results

1) Study of subtype by PCR of subtype-specific reference samples
   The following results were obtained for specimens whose subtype had been determined by virus genome sequencing. Only subtype A specimens were positive, while subtype B, C, and E specimens were all negative, in PCR using primers for the detection of subtype A. Only subtype B specimens were positive, while subtype A,C, and E specimens were all negative, in PCR using primers for the detection of subtype B. Only subtype C specimens were positive, while subtype A, B, and E specimens were all negative, in PCR using primers for the detection of subtype C. Only subtype E specimens were positive, while subtype A, B, and C specimens were all negative, in PCR using primers for the detection of subtype E (Figure 6). All specimens were positive in PCR using primers for amplifying HIV-1 DNA irrespective of subtype (Figure 6).
2) Determination of subtype of HIV patients by PCR
   The HIV-1 subtype was determined in 32 HIV patients who either visited or were hospitalized in Tokyo hospitals. Together with the 11 subtype-specific reference samples, there were 3 subtype A cases, 30 subtype B cases, 2 subtype C cases, and 8 subtype E cases.
   To verify that the above results were correct, the amplified DNA of 21 out of the 43 HIV patients whose subtype was determined by PCR was sequenced and phylogenetically analyzed, giving the results shown in Figure 7. There was complete agreement between the subtypes determined by phylogenetic analysis and the subtypes determined by PCR for these cases of HIV-1.
   The method employed in Example 2 differs significantly from that of Example 1 in that universal primers were used in first PCR, and subtype-specific primers were used in second PCR. As a result, the number of PCR reactions could be reduced to 5/8. In view of the fact that the determination of the subtype had to be postponed in 1 case in Example 1, the method of Example 2 was able to provide more accurate diagnosis and was also simpler.
3) Effect of subtype on testing for drug resistance by genotype
   Drug resistance by genotype was analyzed based on data for subtype B. To investigate whether or not the drug resistance of HIV-1 subtypes other than subtype B could be determined using data for subtype B, the amino acid sequences for HIV-1 protease before and after HAART treatment were determined in 4 patients infected with HIV-1 other than subtype B who were receiving HAART treatment. After HAART treatment in case C3, which was subtype E, amino acid No. 10 had mutated from L (leucine) to F (phenylalanine), and amino acid No. 20 had mutated from K (lysine) to T (threonine). This was recognized as an amino acid mutation indicative of drug resistance in subtype B. However, in all four subjects, amino acid No. 36 was I (isoleucine) from before HAART treatment. In the data for subtype B, HIV-1 with I as the No. 36 amino acid was interpreted as indicative of drug resistance. However, it is difficult to conclude that HIV-1 would have acquired drug resistance before administration of the drug. It would be more logical to view this mutation as irrelevant to drug resistance in HIV-1 subtypes other than subtype B. It may thus be concluded that it is important to diagnose the subtype in advance in order to properly assess drug resistance by genotype.
4) Relationship between subtype and sexual behavior

Figure 9 summarizes the relation between subtype and sexual behavior in 22 patients with HIV who had been interviewed about their sexual preferences. Heterosexuals are those attracted to the opposite sex, while MSM are male homosexuals. About the same number of heterosexuals were subtype B and E, whereas male homosexuals were far more likely to be subtype B. This would seem to indicate that Southeast Asian HIV has spread among heterosexuals but has not spread very much among male homosexuals.

### Example 3

### Subjects and Methodology

1) Western blot-negative and PA-positive serum specimens
   The specimens were 15 serum samples whose blood tests at Tokyo hospitals showed to be HIV-1 negative by Western blotting and HIV-1 positive by PA.
2) Preparation of DNA from plasma RNA
   RNA was prepared using an RNAeasy Kit (QIAGEN) from 200 µL of the aforementioned serum specimen. The RNA was dissolved in pure water and stored at -20°C until immediately before use.
3) Detection of HIV-1 by PCR
   RNA corresponding to 20 µL portions of serum was used as the material, and cDNA was synthesized by 30 minutes of reaction at 42°C using a mixture of primers 12A and 12B with 20 µL reaction solution (10 mM Tris-HCl pH 8.3, 50 mM KCl, 2.5 mM MgCl₂, 1 mM dNTP, 5 µM primer, and 100 unites reverse transcriptase). The cDNA was used as material in nested PCR capable of amplifying the DNA of HIV-1 irrespective of subtype. A mixture of 9AE, 9B, 12A, and 12B was used for the primers in first PCR. A mixture of 10U, 11LB, 11LAE, and 11LC was used for the primers in second PCR.

First PCR was carried out for 30 cycles, where one cycle was 15 seconds at 94°C, 30 seconds at 56°C, and 1 minute at 72°C, with 100 µL reaction solution (10 mM Tris-HCl pH 8.3, 50 mM KCl, 4 mM MgCl₂, 0.2 mM dNTP, 1.0 µM primer, 2.5 units Taq polymerase). Second PCR was carried out for 25 cycles under the same conditions using 2 µL reaction solution from the first PCR. 30 seconds at 60°C was used instead of 30 seconds at 56°C, however.

PCR products (subtype A: 322 bp; subtype B: 358 bp; subtype C: 298 bp; subtype E: 361) were detected by isolation through electrophoresis with 2% agarose gel, and by subsequent ethidium bromide staining.

### Results

No HIV-1 was detected by PCR in any of the 15 serum specimens which were HIV-1 negative by Western blotting and HIV-1 positive by PA (Figure 10). It was proven (Figure 6) that the PCR run here was designed to enable detection of all HIV-1 subtypes. It is highly possible that the inconsistency between the results by Western blotting and PA for the serum specimens tested here is not a problem of subtype, but that the PA method resulted in false positive results.

PCR enabling the detection of HIV-1 irrespective of subtype may therefore be more effective for reliable diagnosis of HIV-1 infection.

The present invention provides a simple method for determining HIV-1 subtypes.

The invention also provides an effective means for determining HIV-1 subtypes.

Sequence ID No. 1 gives the nucleotide sequence for primer 11QA.

Sequence ID No. 2 gives the nucleotide sequence for primer 11BB.

Sequence ID No. 3 gives the nucleotide sequence for primer 11QE.

Sequence ID No. 4 gives the nucleotide sequence for primer 10.

Sequence ID No. 5 gives the nucleotide sequence for primer 12A.

Sequence ID No. 6 gives the nucleotide sequence for primer 12B.

Sequence ID No. 7 gives the nucleotide sequence for primer 12E.

Sequence ID No. 8 gives the nucleotide sequence for primer 9AE.

Sequence ID No. 9 gives the nucleotide sequence for primer 9B.

Sequence ID No. 10 gives the nucleotide sequence for primer 10C.

Sequence ID No. 11 gives the nucleotide sequence for primer 10G.

Sequence ID No. 12 gives the nucleotide sequence for primer 10H.

Sequence ID No. 13 gives the nucleotide sequence for primer 11RC.

Sequence ID No. 14 gives the nucleotide sequence for primer 11RD.

Sequence ID No. 15 gives the nucleotide sequence for primer 11RF.

Sequence ID No. 16 gives the nucleotide sequence for primer 11SG.

Sequence ID No. 17 gives the nucleotide sequence for primer 11SH.

Sequence ID No. 18 gives the nucleotide sequence for primer 11LB.

Sequence ID No. 19 gives the nucleotide sequence for primer 11LE.

Sequence ID No. 20 gives the nucleotide sequence for primer 10U.

Sequence ID No. 21 gives the nucleotide sequence for primer 10KC.

Sequence ID No. 22 gives the nucleotide sequence for primer 10UF.

Sequence ID No. 23 gives the nucleotide sequence for primer 10UG.

Sequence ID No. 24 gives the nucleotide sequence for primer 10UC.

Sequence ID No. 25 gives the nucleotide sequence for primer 11LAE.

Sequence ID No. 26 gives the nucleotide sequence for primer 11LC.

Sequence ID No. 27 gives the nucleotide sequence for primer 11QA1.

Sequence ID No. 28 gives the nucleotide sequence for primer 11VB.

Sequence ID No. 29 gives the nucleotide sequence for primer 11XC.

Sequence ID No. 30 gives the nucleotide sequence for primer 11WE.

Sequence ID No. 31 gives the nucleotide sequence for primer 11TC.

Sequence ID No. 32 gives the nucleotide sequence for primer 11RC1.

Sequence ID No. 33 gives the nucleotide sequence for primer 11SE.

Sequence ID No. 34 gives the nucleotide sequence for primer 11BE.

## Claims

1. A method for determining HIV-1 subtypes, **characterized by** comprising the steps of amplifying nucleic acid using as a target sequence a portion of a nucleotide sequence of the env gene of HIV-1, where at least one of the 5' terminal and 3' terminal nucleotide sequences is different depending on the HIV-1 subtype, and detecting the subtype depending on whether or not the nucleic acid has been amplified.

2. The method according to Claim 1, wherein the target sequence is 100 to 2500 nucleotides long.

3. The method according to Claim 1, wherein the sequence from the 1^{st} through 30^{th} bases from the 3' terminal and/or 5' terminal of the target sequence is different depending on the subtype.

4. The method according to Claim 3, wherein the 3' terminal of the target sequence is in the C3 region of the env gene of HIV-1.

5. The method according to Claim 4, wherein the 5' terminal of the target sequence is in the C2 region of the env gene of HIV-1.

6. The method according to Claim 1, wherein different amplification reactions are carried out using different pairs of primers, and different subtypes are detected.

7. The method according to Claim 6, wherein at least two different subtypes are detected by carrying out amplification at least twice with different pairs of primers using primer pairs consisting of a primer (primer 1) that includes a sequence complementary to a portion of the nucleotide sequence (nucleotide sequence 1) that differs depending on subtype in the C3 region of the env gene of HIV-1, and a primer (primer 2) that includes a sequence complementary to a portion of the nucleotide sequence (nucleotide sequence 2) of the C2 region of the env gene of HIV-1.

8. The method according to Claim 1, wherein a first amplification reaction is carried out with a first pair of primers using as a target sequence a portion of a nucleotide sequence of the env gene of HIV-1, a second amplification reaction is then carried out with a second pair of primers using as a target sequence a portion of said nucleotide sequence, where at least one of the 5' terminal and 3' terminal nucleotide sequences is different depending on the HIV-1 subtype, and the subtype is detected depending on whether or not the nucleic acid has been amplified by the second amplification reaction.

9. The method according to Claim 8, wherein the second pair of primers consists of a primer (primer 1) that includes a sequence complementary to a portion of the nucleotide sequence (nucleotide sequence 1) that differs depending on subtype in the C3 region of the env gene of HIV-1, and a primer (primer 2) that includes a sequence complementary to a portion of the nucleotide sequence (nucleotide sequence 2) of the C2 region of the env gene of HIV-1; and the first pair of primers consists of a primer (primer 3) that includes a sequence complementary to a portion of a nucleotide sequence (nucleotide sequence 3) of a region downstream of the 3' terminal of nucleotide sequence 1 of the env gene of HIV-1, and a primer (primer 4) that includes a sequence complementary to a portion of a nucleotide sequence (nucleotide sequence 4) of a region upstream of the 5' terminal of nucleotide sequence 2 of the env gene of HIV-1.

10. The method according to Claim 8, wherein at least two subtypes are distinguished by repeating at least once, with different pairs of second primers, a series of operations comprising: a first amplification reaction that is carried out with the first pair of primers using as a target sequence a portion of a nucleotide sequence of the env gene of HIV-1; a second amplification reaction that is then carried out with the second pair of primers using as a target sequence a nucleotide sequence within said target sequence; and the detection of subtypes depending on whether or not the nucleic acid has been amplified by the second amplification reaction.

11. The method according to Claim 10, wherein subtypes A, B, C, and E are distinguished by:
(a) detecting subtype A using as the first primer pair a mixture of primer 12A containing nucleotide sequence GCAATAGAAAAATTCTCCTC (Sequence ID No. 5) and primer 12B containing nucleotide sequence ACAGTAGAAAAATTCCCCTC (Sequence ID No. 6), and a mixture of primer 9AE containing nucleotide sequence CACAGTACAATGCACACATG (Sequence ID No. 8) and primer 9B containing nucleotide sequence CACAGTACAATGTACACATG (Sequence ID No. 9), and using as the second primer pair primer 11QA1 containing nucleotide sequence CTCCTGAGGAGTTAGCAAAG (Sequence ID No. 27) and primer 10U containing nucleotide sequence CTGTTAAATGGCAGTCTAGC (Sequence ID No. 20);
(b) detecting subtype B using as the first primer pair a mixture of primer 12A containing nucleotide sequence GCAATAGAAAAATTCTCCTC (Sequence ID No. 5) and primer 12B containing nucleotide sequence ACAGTAGAAAAATTCCCCTC (Sequence ID No. 6), and a mixture of primer 9AE containing nucleotide sequence CACAGTACAATGCACACATG (Sequence ID No. 8) and primer 9B containing nucleotide sequence CACAGTACAATGTACACATG (Sequence ID No. 9), and using as the second primer pair primer 11VB containing nucleotide sequence CACAATTAAAACTGTGCATTAC (Sequence ID No. 28) and primer 10U containing nucleotide sequence CTGTTAAATGGCAGTCTAGC (Sequence ID No. 20);
(c) detecting subtype C using as the first primer pair a mixture of primer 12A containing nucleotide sequence GCAATAGAAAAATTCTCCTC (Sequence ID No. 5) and primer 12B containing nucleotide sequence ACAGTAGAAAAATTCCCCTC (Sequence ID No. 6), and a mixture of primer 9AE containing nucleotide sequence CACAGTACAATGCACACATG (Sequence ID No. 8) and primer 9B containing nucleotide sequence CACAGTACAATGTACACATG (Sequence ID No. 9), and using as the second primer pair primer 11XC containing nucleotide sequence TTGTTTTATTAGGGAAGTGTTC (Sequence ID No. 29) and primer 10UC containing nucleotide sequence CTGTTAAATGGTAGTCTAGC (Sequence ID No. 24); and
(d) detecting subtype E using as the first primer pair a mixture of primer 12A containing nucleotide sequence GCAATAGAAAAATTCTCCTC (Sequence ID No. 5) and primer 12B containing nucleotide sequence ACAGTAGAAAAATTCCCCTC (Sequence ID No. 6), and a mixture of primer 9AE containing nucleotide sequence CACAGTACAATGCACACATG (Sequence ID No. 8) and primer 9B containing nucleotide sequence CACAGTACAATGTACACATG (Sequence ID No. 9), and using as the second primer pair primer 11WE containing nucleotide sequence CTCTACAATTAAAATGATGCATTG (Sequence ID No. 30) and primer 10U containing nucleotide sequence CTGTTAAATGGCAGTCTAGC (Sequence ID No. 20).

12. The method according to Claim 8, wherein at least two subtypes are distinguished by repeating at least once, with different pairs of first and second primers, a series of operations comprising: a first amplification reaction that is carried out with a first pair of primers using as a target sequence a portion of a nucleotide sequence of the env gene of HIV-1; a second amplification reaction that is then carried out with a second pair of primers using as a target sequence a nucleotide sequence within said target sequence; and the detection of subtypes depending on whether or not the nucleic acid has been amplified by the second amplification reaction.

13. The method according to Claim 12, wherein subtypes A, B, and E are distinguished by:
(a) detecting subtype A using as the first primer pair primer 12A containing nucleotide sequence GCAATAGAAAAATTCTCCTC (Sequence ID No. 5) and primer 9AE containing nucleotide sequence CACAGTACAATGCACACATG (Sequence ID No. 8), and using as the second primer pair primer 11QA containing nucleotide sequence CTCCTGAGGGGTTAGCAAAG (Sequence ID No. 1) and primer 10 containing nucleotide sequence AAATGGCAGTCTAGCAGAAG (Sequence ID No. 4);
(b) detecting subtype B using as the first primer pair primer 12B containing nucleotide sequence ACAGTAGAAAAATTCCCCTC (Sequence ID No. 6) and primer 9B containing nucleotide sequence CACAGTACAATGTACACATG (Sequence ID No. 9), and using as the second primer pair primer 11BB containing nucleotide sequence CTGTGCATTACAATTTCTGG (Sequence ID No. 2) and primer 10 containing nucleotide sequence AAATGGCAGTCTAGCAGAAG (Sequence ID No. 4); and
(c) detecting subtype E using as the first primer pair primer 12E containing nucleotide sequence GCAATAGAAAAATTCCCCTC (Sequence ID No. 7) and primer 9AE containing nucleotide sequence CACAGTACAATGCACACATG (Sequence ID No. 8), and using as the second primer pair primer 11QE containing nucleotide sequence CTCCTGAGGGTGGTTGAAAG (Sequence ID No. 3) and primer 10 containing nucleotide sequence AAATGGCAGTCTAGCAGAAG (Sequence ID No. 4).

14. The method according to Claim 1, further comprising the steps of amplifying nucleic acid using as a target sequence a portion of a nucleotide sequence of the HIV-1 genome, the nucleotide sequence being highly conserved among all subtypes, and ascertaining the presence or absence of HIV-1 depending on whether or not the nucleic acid has been amplified.

15. The method according to Claim 14, wherein the step for ascertaining the presence or absence of HIV-1 comprises amplifying the nucleic acid with a first primer pair using as a target sequence a portion of a nucleotide sequence of the HIV-1 genome, the nucleotide sequence being highly conserved among all subtypes, then carrying out a second amplifying reaction with a second primer pair using as a target sequence a nucleotide sequence in said target sequence, and ascertaining the presence or absence of HIV-1 depending on whether or not the nucleic acid has been amplified.

16. The method according to Claim 15, wherein the primers that are used comprise a mixture of a plurality of upstream primers with differing nucleotide sequences and a plurality of downstream primers with differing nucleotide sequences.

17. The method according to Claim 16, wherein the first primers comprise a mixture of primer 12A containing nucleotide sequence GCAATAGAAAAATTCTCCTC (Sequence ID No. 5), primer 12B containing nucleotide sequence ACAGTAGAAAAATTCCCCTC (Sequence ID No. 6), primer 9AE containing nucleotide sequence CACAGTACAATGCACACATG (Sequence ID No. 8), and primer 9B nucleotide sequence CACAGTACAATGTACACATG (Sequence ID No. 9), and the second primer pair comprises primer 11LB containing nucleotide sequence AATTTCTGGGTCCCCTCCTG (Sequence ID No. 18), primer 11LAE containing nucleotide sequence AATTTCTAGATCCCCTCCTG (Sequence ID No. 25), primer 11LC containing nucleotide sequence AATTTCTAGGTCCCCTCCTG (Sequence ID No. 26), and primer 10U containing nucleotide sequence CTGTTAAATGGCAGTCTAGC (Sequence ID No. 20).

18. A kit for determining HIV-1 subtypes, comprising primer pairs in which a target sequence is a portion of a nucleotide sequence of the env gene of HIV-1, where at least one of the 5' terminal and 3' terminal nucleotide sequences is different depending on the subtype.
